# EUROPÄISCHE PATENTSCHRIFT

(11) **EP 2 349 983 B1**
(45) Veröffentlichungstag und Bekanntmachung des Hinweises auf die Patenterteilung: **14.09.2016**
(21) Anmeldenummer: 09744090.3
(22) Anmeldetag: 02.11.2009
(51) Int. Cl.: C07C 243/38, A61K 31/166

(54) **DIFLUORPHENYL-DIACYLHYDRAZID-DERIVATE**
DIFLUORPHENYL DIACYLHYDRAZIDE DERIVATES
DÉRIVÉS DE DIFLUOROPHÉNYL-DIACYLHYDRAZIDE

(30) Priorität: 26.11.2008 DE 102008059133
(43) Veröffentlichungstag der Anmeldung: 03.08.2011
(73) Patentinhaber: Merck Patent GmbH, 64293 Darmstadt (DE)
(72) Erfinder: FUCHSS, Thomas, 64625 Bensheim-Auerbach (DE); GRAEDLER, Ulrich, 69469 Weinheim (DE); BEIER, Norbert, 64354 Reinheim (DE); GERICKE, Rolf, 64342 Seeheim-Jugenheim (DE); LANG, Florian, 72076 tuebingen (DE)
(86) Internationale Anmeldenummer: PCT/EP2009/007827
(87) Internationale Veröffentlichungsnummer: WO 2010/060522

(56) Entgegenhaltungen:
- WO-A1-2006/105850
- WO-A1-2007/093264
- WO-A1-2009/103484

## Beschreibung

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen, bei denen die Hemmung, Regulierung und/oder Modulation der Signaltransduktion von Kinasen, insbesondere der zellvolumenregulierten humanen Kinase h-sgk (human serum and glucocorticoid dependent kinase oder SGK) eine Rolle spielt, ferner pharmazeutische Zusammensetzungen, die diese Verbindungen enthalten, sowie die Verwendung der Verbindungen zur Behandlung SGKbedingter Krankheiten.

Die SGK mit den Isoformen SGK-1, SGK-2 und SGK-3 sind eine Serin/Threonin-Proteinkinase Familie (WO 02/17893).
Die erfindungsgemäßen Verbindungen sind vorzugsweise selektive Inhibitoren der SGK-1. Ferner können sie Inhibitoren der SGK-2 und/oder SGK-3 sein.

Im einzelnen betrifft die vorliegende Erfindung Verbindungen, die die Signaltransduktion der SGK hemmen, regulieren und/oder modulieren, Zusammensetzungen, die diese Verbindungen enthalten, sowie Verfahren zu ihrer Verwendung zur Behandlung von SGK-bedingten Krankheiten und Leiden wie Diabetes (z.B. Diabetes mellitus, diabetische Nephropathie, diabetische Neuropathie, diabetische Angiopathie und Mikroangiopathie), Fettsucht, metabolisches Syndrom (Dyslipidämie), systemische und pulmonale Hypertonie, Herzkreislauferkrankungen (z.B. kardiale Fibrosen nach Myokardinfarkt, Herzhypertrophie und Herzinsuffizienz, Arteriosklerose) und Nierenerkrankungen (z.B. Glomerulosklerose, Nephrosklerose, Nephritis, Nephropathie, Störung der Elektrolytausscheidung), allgemein bei jeglicher Art von Fibrosen und entzündlichen Prozessen (z.B. Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer).
Die erfindungsgemäßen Verbindungen können auch das Wachstum von Tumorzellen und Tumormetastasen hemmen und sind deshalb für die Tumortherapie geeignet.
Die erfindungsgemäßen Verbindungen finden auch Verwendung bei der Behandlung von peptischen Ulcera, insbesondere bei Formen, die durch Stress ausgelöst werden.
Die erfindungsgemäßen Verbindungen finden weiterhin Verwendung zur Behandlung von Koagulopathien, wie z.B. Dysfibrinogenämie, Hypoprokonvertinämie, Hämophilie B, Stuart-Prower-Defekt, ProthrombinKomplex-Mangel, Verbrauchskoagulopathie, Hyperfibrinolyse, Immunokoagulopathie oder komplexer Koagulopathien, wie auch bei neuronaler Erregbarkeit, z.B. Epilepsie. Die erfindungsgemäßen Verbindungen können auch bei der Behandlung eines Glaukoms oder Katarakt therapeutisch eingesetzt werden.
Die erfindungsgemäßen Verbindungen finden ferner Verwendung bei der Behandlung bakterieller Infektionen sowie in einer antiinfektiösen Therapie. Die erfindungsgemäßen Verbindungen können auch zur Steigerung der Lernfähigkeit und Aufmerksamkeit therapeutisch eingesetzt werden. Darüberhinaus wirken die erfindungsgemäßen Verbindungen der Zellalterung und Stress entgegen und steigern somit die Lebenserwartung und die Fitness im Alter.
Die erfindungsgemäßen Verbindungen finden ferner Verwendung bei der Behandlung von Tinitus.

Die Identifikation von kleinen Verbindungen, die die Signaltransduktion der SGK spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.
Insbesondere zeigen sie inhibierende Eigenschaften der SGK.

Die erfindungsgemäßen Verbindungen können darüber hinaus auch zur Behandlung von Autoimmunerkrankungen, inflammatorischen und proliferativen Erkrankungen, AIDS, Asthma, Rhinitis und Morbus Crohn verwendet werden.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer-(HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemolumineszenz nachweisbar (Ross et al., Biochem. J., 2002, 366, 977-981).

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschte Zellen mehr im Körper nachgewiesen werden.

### STAND DER TECHNIK

Andere Acylhydrazidderivate sind in der WO 2006/105850 und in der WO 2007/093264 als SGK Inhibitoren beschrieben.

In der WO 00/62781 ist die Verwendung von Arzneimitteln enthaltenden Hemmstoffen der zellvolumenregulierten humanen Kinase hSGK beschrieben. Die Verwendung von Kinase-Inhibitoren in der antiinfektiösen Therapie ist von C. Doerig in Cell. Mol. Biol. Lett. 2003, 8,(2A):524-525 beschrieben.
Die Verwendung von Kinase-Inhibitoren bei Fettsucht ist von N.Perrotti in J. Biol. Chem. 2001, 276(12):9406-9412 beschrieben.

In nachstehenden Literaturstellen wird die Verwendung von SGK-Hemmern bei der Behandlung von Krankheiten nahegelegt und/oder beschrieben:
1: Chung EJ, Sung YK, Farooq M, Kim Y, Im S, Tak WY, Hwang YJ, Kim Yl, Han HS, Kim JC, Kim MK. Gene expression profile analysis in human hepatocellular carcinoma by cDNA microarray. Mol. Cells. 2002;14:382-7.
2: Brickley DR, Mikosz CA, Hagan CR, Conzen SD. Ubiquitin modification of serum and glucocorticoid-induced protein kinase-1(SGK-1). J. Biol. Chem. 2002; 277:43064-70.
3: Fillon S, Klingel K, Warntges S, Sauter M, Gabrysch S, Pestel S, Tanneur V, Waldegger S, Zipfel A, Viebahn R, Haussinger D, Broer S, Kandolf R, Lang F. Expression of the serine/threonine kinase hSGK1 in chronic viral hepatitis. Cell Physiol Biochem. 2002; 12:47-54.
4: Brunet A, Park J, Tran H, Hu LS, Hemmings BA, Greenberg ME. Protein kinase SGK mediates survival signals by phosphorylating the forkhead transcription factor FKHRL1 (FOXO3a). Mol. Cell. Biol. 2001; 21:952-65
5: Mikosz CA, Brickley DR, Sharkey MS, Moran TW, Conzen SD. Glucocorticoid receptor-mediated protection from apoptosis is associated with induction of the serine/threonine survival kinase gene, sgk-1. J. Biol. Chem. 2001; 276:16649-54.
6: Zuo Z, Urban G, Scammell JG, Dean NM, McLean TK, Aragon I, Honkanen RE. Ser/Thr protein phosphatase type 5 (PP5) is a negative regulator of glucocorticoid receptor-mediated growth arrest. Biochemistry 1999; 38:8849-57.
7: Buse P, Tran SH, Luther E, Phu PT, Aponte GW, Firestone GL. Cell cycle and hormonal control of nuclear-cytoplasmic localization of the serum- and glucocorticoid-inducible protein kinase, Sgk, in mammary tumor cells. A novel convergence point of anti-proliferative and proliferative cell signalling pathways. J. Biol. Chem. 1999; 274:7253-63.
8: M. Hertweck, C. Göbel, R. Baumeister: C. elegans SGK-1 is the critical component in the Akt/PKB Kinase complex to control stress response and life span. Developmental Cell 2004, 6:577-588

### ZUSAMMENFASSUNG DER ERFINDUNG

Die Erfindung betrifft Verbindungen der Formel I 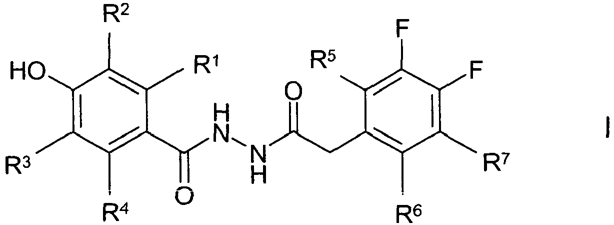 worin
- R¹, R⁴, R⁵: jeweils unabhängig voneinander H, Hal, A oder CN,
- R², R³: jeweils unabhängig voneinander H, Hal oder A,
- R⁶, R⁷: jeweils unabhängig voneinander H, A, OA, NHA oder NA₂,
- A: Alkyl mit 1-6 C-Atomen, worin 1-5 H-Atome durch F ersetzt sein können oder
Cycloalkyl mit 3-7 C-Atomen,
- Hal: F, Cl, Br oder I,
sowie ihre pharmazeutisch verwendbaren Salze und Stereoisomere, einschließlich deren Mischungen in allen Verhältnissen.

Gegenstand der Erfindung sind die Verbindungen der Formel I und ihre Salze sowie ein Verfahren zur Herstellung von Verbindungen der Formel I sowie ihrer pharmazeutisch verwendbaren Salze und Stereoisomere, dadurch gekennzeichnet, daß man
a) eine Verbindung der Formel II 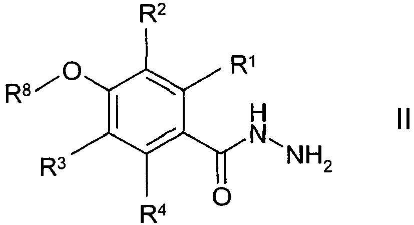 worin
   R¹, R², R³, R⁴ die in Anspruch 1 angegebenen Bedeutungen haben und
   R8 eine Hydroxyschutzgruppe bedeutet
   mit einer Verbindung der Formel III 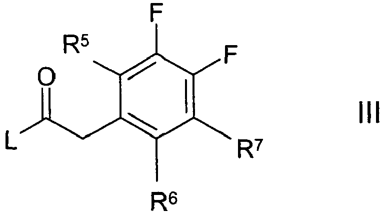 worin
   L Cl, Br, I oder eine freie oder reaktionsfähig funktionell abgewandelte OH-Gruppe bedeutet und
   R⁵, R⁶ und R⁷ die in Anspruch 1 angegebenen Bedeutungen haben,
   umsetzt, und anschließend R⁸ abspaltet,
   oder
b) eine Verbindung der Formel IV 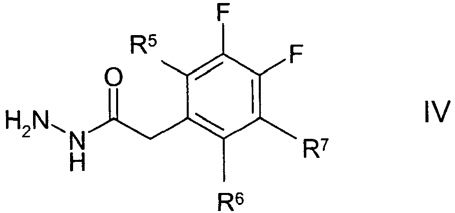 worin
   R⁵, R⁶ und R⁷ die in Anspruch 1 angegebenen Bedeutungen haben,
   mit einer Verbindung der Formel V 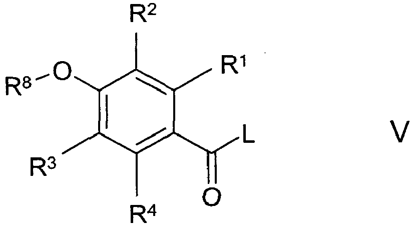 worin
   L Cl, Br, I oder eine freie oder reaktionsfähig funktionell abgewandelte OH-Gruppe bedeutet und
   R¹, R², R³ und R⁴ die in Anspruch 1 angegebenen Bedeutungen haben und
   R⁸ eine Hydroxyschutzgruppe bedeutet,
   umsetzt, und anschließend R⁸ abspaltet
   oder
c) sie aus einem ihrer funktionellen Derivate durch Behandeln mit einem solvolysierenden oder hydrogenolysierenden Mittel in Freiheit setzt,
und/oder eine Base oder Säure der Formel I in eines ihrer Salze umwandelt.

Unter den Verbindungen der Formel I versteht man auch die Hydrate und Solvate dieser Verbindungen, ferner pharmazeutisch verwendbare Derivate. Gegenstand der Erfindung sind auch die Stereoisomeren (E, Z-Isomeren) sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 1995, 115, 61-67 beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung sind auch Mischungen der erfindungsgemäßen Verbindungen der Formel I, z.B. Gemische zweier Diastereomerer oder Enantiomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen, insbesondere liegen die erfindungsgemäßen Verbindungen als Racemat vor.

Für alle Reste, die mehrfach auftreten, gilt, daß deren Bedeutungen unabhängig voneinander sind.
Vor- und nachstehend haben die Reste bzw. Parameter R¹, R², R³, R⁴, R⁵, R⁶ und R⁷ die bei der Formel I angegebenen Bedeutungen, falls nicht ausdrücklich etwas anderes angegeben ist.

A bedeutet Alkyl, ist unverzweigt (linear) oder verzweigt, und hat 1, 2, 3, 4, 5, oder 6 C-Atome. A bedeutet vorzugsweise Methyl, weiterhin Ethyl, Propyl, Iso-propyl, Butyl, Isobutyl, sek.-Butyl oder tert.-Butyl, ferner auch Pentyl, 1-, 2-oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1, 2,2-Trimethylpropyl, weiter bevorzugt z.B. Trifluormethyl.
A bedeutet weiterhin Cycloalkyl mit 3-7 C-Atomen, vorzugsweise Cyclopentyl oder Cyclohexyl.

R¹ und R² bedeuten vorzugsweise A, besonders bevorzugt, jeweils unabhängig voneinander Methyl, Ethyl, Propyl, Isopropyl oder Butyl.
R³ und R⁴ bedeuten vorzugsweise H.
R⁵ bedeutet vorzugsweise H.

R⁶ und R⁷ bedeuten vorzugsweise, jeweils unabhängig voneinander H oder OA, besonders bevorzugt, jeweils unabhängig voneinander, H, Methoxy, Ethoxy, Propoxy oder Isopropoxy.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis If ausgedrückt werden, die der Formel I entsprechen und worin die nicht näher bezeichneten Reste die bei der Formel I angegebene Bedeutung haben, worin jedoch

| | | |
|---|---|---|
| in Ia | R¹, R² | A bedeuten; |
| in Ib | R³, R⁴ | H bedeuten; |
| in Ic | R⁵ | H bedeutet; |
| in Id | R⁶, R⁷ | jeweils unabhängig voneinander H oder OA |
| | | bedeuten; |
| in Ie | A | Methyl, Ethyl, Propyl oder Isopropyl, |
| | | bedeutet; |
| in If | R¹, R² | A, |
| | R³, R⁴ | H, |
| | R⁵ | H, |
| | R⁶ , R⁷ | jeweils unabhängig voneinander H oder OA, |
| | A | Alkyl mit 1-6 C-Atomen, worin 1-5 H-Atome durch F ersetzt sein können, |
| | Hal | F, Cl, Br oder I |
| | | bedeuten; |

sowie ihre pharmazeutisch verwendbaren Salze und Stereoisomere, einschließlich deren Mischungen in allen Verhältnissen.

Die erfindungsgemäßen Verbindungen und auch die Ausgangsstoffe zu ihrer Herstellung werden im übrigen nach an sich bekannten Methoden hergestellt wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den erfindungsgemäßen Verbindungen umsetzt.
Die Ausgangsverbindungen sind in der Regel bekannt. Sind sie neu, so können sie aber nach an sich bekannten Methoden hergestellt werden.

Verbindungen der Formel I können vorzugsweise erhalten werden, indem man ein Hydrazid der Formel II mit einer Verbindung der Formel III umsetzt.
Die Umsetzung erfolgt nach Methoden, die dem Fachmann bekannt sind. Die Umsetzung erfolgt in der Regel in einem inerten Lösungsmittel, gegebenenfalls in Gegenwart eines säurebindenden Mittels vorzugsweise einer organischen Base wie DIPEA, Triethylamin, Dimethylanilin, Pyridin, Chinolin oder DBU oder eines Überschusses der Hydrazidkomponente der Formel II.

Die Hydroxyschutzgruppe R⁸ bedeutet vorzugsweise Benzyl, Allyl, Benzyloxymethyl, tert.-Butyldimethylsilyl (TBDMS), tert.-Butyldiphenylsilyl (TBDPS), 3,4-Dimethoxybenzyl, p-Methoxybenzyl, 2-Methoxyethoxymethyl (MEM), Methoxymethyl (MOM), Tetrahydropyran-2-yl (THP) oder 2-(Trimethylsilyl)-ethoxymethyl (SEM).

Als inerte Lösungsmittel eignen sich z.B. Kohlenwasserstoffe wie Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Ethanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist DMF.

Die Umsetzung erfolgt in der Regel in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin, Chinolin oder DBU kann günstig sein.

In den Verbindungen der Formel III bedeutet L vorzugsweise Cl, Br, I oder eine freie oder eine reaktionsfähig abgewandelte OH-Gruppe, wie z.B. ein aktivierter Ester (z.B. ein Pentafluorphenyl- oder N-Hydroxybenzotriazolester), ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy) oder durch Umsetzung mit Carbodiimiden (z.B. DAPECI) oder Uroniumsalzen und ihren Abkömmlingen (z.B. TOTU, ByPOP).
Aktivierte Ester werden zweckmäßig in situ gebildet, wobei für deren Umsetzung gegebenenfalls Zusätze von HOBt, HOOBt oder N-Hydroxysuccinimid günstig sind.

Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart) beschrieben.

Verbindungen der Formel I können weiterhin vorzugsweise erhalten werden, indem man ein Hydrazid der Formel IV mit einer Verbindung der Formel V umsetzt.

Die Umsetzung erfolgt in der Regel in einem inerten Lösungsmittel, in Gegenwart eines säurebindenden Mittels vorzugsweise einer organischen Base wie DIPEA, Triethylamin, Dimethylanilin, Pyridin,Chinolin oder DBU oder eines Überschusses der Hydrazidkomponente der Formel IV

Als inerte Lösungsmittel eignen sich die oben genannten.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.
Die Reaktionszeit liegt je nach den angewendeten Bedingungen zwischen einigen Minuten und mehreren Tagen, die Reaktionstemperatur zwischen etwa -30° und 140°, normalerweise zwischen -10° und 90°, insbesondere zwischen etwa 0° und etwa 70°.

In den Verbindungen der Formel IV bedeutet L vorzugsweise Cl, Br, I oder eine freie oder eine reaktionsfähig abgewandelte OH-Gruppe, wie z.B. ein aktivierter Ester( z.B. ein Pentafluorphenyl- oder N-Hydroxybenzotriazolester), ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy) oder durch Umsetzung mit Carbodiimiden (z.B. DAPECI) oder Uroniumsalzen und ihren Abkömmlingen (z.B. TOTU, ByPOP).

In den Verbindungen der Formel V hat R⁸ die oben genannten bevorzugten Bedeutungen.

Verbindungen der Formel I können ferner erhalten werden, indem man Verbindungen der Formel I aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer Gruppe -COOH oder OH eine Gruppe -COOR" oder OR" tragen, worin R" eine Hydroxyschutzgruppe bedeutet. Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.
Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, Allyl, Benzyloxymethyl, tert.-Butyldimethylsilyl (TBDMS), tert.-Butyldiphenylsilyl (TBDPS), 3,4-Dimethoxybenzyl, p-Methoxybenzyl, 2-Methoxyethoxymethyl (MEM), Methoxymethyl (MOM), Tetrahydropyran-2-yl (THP) oder 2-(Trimethylsilyl)-ethoxymethyl (SEM).

Das in-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5M HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ, Benzyl) können z. B. durch Behandeln mit Wasserstoff oder mit Ammoniumformiat (anstelle von Wasserstoffgas) in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF oder Ether wie Dioxan oder Tetrahydrofuran. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der Benzyl-Gruppe gelingt z.B. an 5 bis 10 %igem Pd/C in Tetrahydrofuran in einer Wasserstoffatmosphäre unter Normalbedingungen.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Trifluormethylbenzol, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Ester können z.B. mit Essigsäure oder mit NaOH oder KOH in Wasser, Wasser-THF oder Wasser-Dioxan bei Temperaturen zwischen 0 und 100° verseift werden.

Die Spaltung eines Ethers erfolgt unter Methoden, wie sie dem Fachmann bekannt sind.
Eine Standardmethode zur Etherspaltung, z.B. eines Methylethers, ist die Verwendung von Bortribromid.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, lodid, isethionat, isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer lonenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkyl-halogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl Wasserals auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine erfindungsgemäße Verbindung und/oder ihre pharmazeutisch verwendbaren Salze und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glycerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die erfindungsgemäßen Verbindungen sowie deren Salze lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Verbindungen sowie die Salze können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können bispielsweise für die orale Verabreichung geeignete Formulierungen Geschmacksstoffe enthalten.

Eine therapeutisch wirksame Menge einer Verbindung der vorliegenden Erfindung hängt von einer Reihe von Faktoren ab, einschließlich z.B. dem Alter und Gewicht des Menschen oder Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine erfindungsgemäße Verbindung und/oder ihre pharmazeutisch verwendbaren Salze und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer erfindungsgemäßen Verbindung und/oder ihrer pharmazeutisch verwendbaren Salze und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer erfindungsgemäßen Verbindung und/oder ihrer pharmazeutisch verwendbaren Salze und Stereoisomere, einschließlich deren Mischungen in allen Verhältnissen, und einer wirksamen Menge eines weiteren Arzneimittelwirkstoffs gelöst oder in lyophylisierter Form vorliegt.

### VERWENDUNG

Die vorliegenden Verbindungen eignen sich als pharmazeutische Wirkstoffe für Säugetiere, insbesondere für den Menschen, bei der Behandlung von SGK-bedingten Krankheiten.

Gegenstand der Erfindung ist somit die Verwendung von Verbindungen nach Anspruch 1, sowie ihrer pharmazeutisch verwendbaren Derivate, Solvate und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt.
Bevorzugt ist hierbei SGK.

Bevorzugt ist die Verwendung von Verbindungen gemäß Anspruch 1, sowie ihrer pharmazeutisch verwendbaren Derivate, Solvate und Stereoisomere, einschließlich deren Mischungen in allen Verhältnissen, zur Herstellung eines Arzneimittels zur Behandlung von Krankheiten, die durch inhibierung der SGK durch die Verbindungen nach Anspruch 1 beeinflußt werden.

Die vorliegende Erfindung umfasst die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung von Diabetes (z.B. Diabetes mellitus, diabetische Nephropathie, diabetische Neuropathie, diabetische Angiopathie und Mikroangiopathie), Fettsucht, metabolisches Syndrom (Dyslipidämie), systemische und pulmonale Hypertonie, Herzkreislauferkrankungen (z.B. kardiale Fibrosen nach Myokardinfarkt, Herzhypertrophie und Herzinsuffizienz, Arteriosklerose) und Nierenerkrankungen (z.B. Glomerulosklerose, Nephrosklerose, Nephritis, Nephropathie, Störung der Elektrolytausscheidung), allgemein bei jeglicher Art von Fibrosen und entzündlichen Prozessen (z.B. Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer).
Die erfindungsgemäßen Verbindungen können auch das Wachstum von Krebs, Tumorzellen und Tumormetastasen hemmen und sind deshalb für die Tumortherapie geeignet.
Die erfindungsgemäßen Verbindungen finden weiterhin Verwendung zur Behandlung von Koagulopathien, wie z.B. Dysfibrinogenämie, Hypoprokonvertinämie, Hämophilie B, Stuart-Prower-Defekt, ProthrombinKomplex-Mangel, Verbrauchskoagulopathie, Hyperfibrinolyse, Immunokoagulopathie oder komplexer Koagulopathien, wie auch bei neuronaler Erregbarkeit, z.B. Epilepsie. Die erfindungsgemäßen Verbindungen können auch bei der Behandlung eines Glaukoms oder Katarakt therapeutisch eingesetzt werden.
Die erfindungsgemäßen Verbindungen finden ferner Verwendung bei der Behandlung bakterieller Infektionen sowie in einer antiinfektiösen Therapie. Die erfindungsgemäßen Verbindungen können auch zur Steigerung der Lernfähigkeit und Aufmerksamkeit therapeutisch eingesetzt werden.

Bevorzugt ist die Verwendung von Verbindungen gemäß Anspruch 1, sowie ihrer pharmazeutisch verwendbaren Derivate, Solvate und Stereoisomere, einschließlich deren Mischungen in allen Verhältnissen, zur Herstellung eines Arzneimittels zur Behandlung oder Vorbeugung von Diabetes, Fettsucht, metabolischem Syndrom (Dyslipidämie), systemischer und pulmonaler Hypertonie, Herzkreislauferkrankungen und Nierenerkrankungen, allgemein bei jeglicher Art von Fibrosen und entzündlichen Prozessen, Krebs, Tumorzellen, Tumormetastasen, Koagulopathien, neuronaler Erregbarkeit, Glaukom, Katarakt, bakteriellen Infektionen sowie in einer antiinfektiösen Therapie, zur Steigerung der Lernfähigkeit und Aufmerksamkeit, sowie zur Behandlung und Prophylaxe von Zellalterung und Stress.

Bei Diabetes handelt es sich vorzugsweise um Diabetes mellitus, diabetische Nephropathie, diabetische Neuropathie, diabetische Angiopathie und Mikroangiopathie.

Bei Herzkreislauferkrankungen handelt es sich vorzugsweise um kardiale Fibrosen nach Myokardinfarkt, Herzhypertrophie, Herzinsuffizienz und Arteriosklerose.

Bei Nierenerkrankungen handelt es sich vorzugsweise um Glomerulosklerose, Nephrosklerose, Nephritis, Nephropathie und Störung der Elektrolytausscheidung.

Bei Fibrosen und entzündlichen Prozessen handelt es sich vorzugsweise um Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer.

### ASSAYS

Die in den Beispielen beschriebenen erfindungsgemäßen Verbindungen wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).
Die Hemmung der SGK1 Proteinkinase kann im Filterbindungsverfahren bestimmt werden.

### Zellassay

HeLa-Zellen werden mit einer Dichte von 10 - 20 x 10³ Zellen/cm² in 6-Well-MTPs (Costar Corning, # 3506) in DMEM-Medium, ergänzt mit 10 % fötalem Kälberserum (FCS), 2 mM Glutamin und 1 mM Natriumpyruvat, ausplattiert. Nach 24 Std. bei 37 °C und 5 % CO₂ in einem Zellinkubator wird jeder Well des Weiteren mit 25 µl einer 100X DMSO-Lösung der Verbindung ergänzt; diese Lösung wird im Überstand der Zellkultur 100fach verdünnt, was zu der erwarteten SGK1-Inhibitorkonzentration bei einer 1 %igen DMSO-Konzentration führt. Die Zellen werden weitere 24 Std. unter den gleichen Bedingungen inkubiert.
Anschließend werden die Überstände entfernt (durch Absaugen) und die Zellen werden einmal mit 1 ml/Well eiskalter phosphatgepufferter Kochsalzlösung (PBS) gewaschen. Zu jedem Well werden 250 µl des eiskalten Lysepuffers (50 mM Tris/HCl, 1 mM EDTA, 1mM EGTA, 0,5 mM aktiviertes Na₃VO₄, 10 mM Glycerophosphat, 50 mM NaF, 5 mM Na-Pyrophosphat, 1 % Triton X100, 1 mM DTT, 0,1 mM PMSF und 1 µM Microcystin und jeweils 1 µg/ml Aprotinin, Pepstatin bzw. Leupeptin) zugegeben. Die Zellen werden vom Boden des Wells heruntergekratzt und die Zellsuspension wird mehrere Male mit einer Eppendorf-Pipette aufgesaugt; dadurch werden die Zellen lysiert und homogenisiert. Die Zelllysate (250 µl/Vial) werden in vorgekühlte Eppendorf-Vials (bei -24 °C) überführt. Die Zellsuspensionen werden 1 - 2 sec lang mit Ultraschall behandelt; die Zelllysate werden mit flüssigem Stickstoff schockgefroren und bei -24 °C gelagert.
16 µl Aliquots der Zelllysate werden in 6 µl des 4 X NuPage^{®} LDS-Probenpuffers plus 1 µl β-Mercaptoethanol überführt und 10 min lang bei 70 °C erhitzt. Für den Nachweis der P-NDRG1- und NDRG1-Spiegel werden 20-µl-Aliquots der Proben auf ein NuPage^{®} -SDS-Gel (4 - 12 % Bis-Tris-Gel (für den P-NDRG1-Nachweis) oder 7 % Bis-Tris-Gel (für den Nachweis von NDRG1)) geladen und entsprechend der Proteingröße getrennt. Die Proteinbanden werden elektrophoretisch auf 0,2 µm Nitrocellulosemembranen überführt und unter Verwendung von NDRG1-bzw. NDRG1-Phospho-Thrx3-Antiseren, bei einer Konzentration von 1 µg/ml einem Immunoblot unterzogen. Beide Antiseren wurden von Prof. Sir Phil Cohen, Division of Signal Transduction Therapy, Universität Dundee, Schottland, erhalten. Für den Nachweis von P-NDRG1 wurden 10 µg/ml des nichtphosphorylierten Nona-Peptids RSRSHTSEG zum Inkubationspuffer gegeben.

Die Bindung des primären Antikörpers wurde unter Verwendung von an Kaninchen-Peroxidase konjugiertem Antischaf-IG-Antikörper (1:5000-Verdünnung, Calbiochem), gefolgt von verstärkter Chemilumineszenz (SuperSignal West Dura Extended Duration, Pierce) nachgewiesen. Der P-NDRG1-Spiegel wird auf den NDRG1-Spiegel in den Proben normiert wiedergegeben. Die NDRG1-Spiegel werden nach dem Strippen der Nitrocellulosemembranen unter Verwendung des RestoreTM Western Blot Stripping-Puffers und dem Verfahren von Pierce bestimmt.
Bei Verwendung des P-NDRG1-Antiserums kann eine Abnahme des Phosphorylierungsspiegels des NDRG1-Proteins einfach nachgewiesen werden. Die Abnahme der Intensität der Bande im Western Blot bei Verwendung des P-NDRG1-Antiserums wird bestimmt, in einem halblogarithmischen Diagramm gegen die Zellkulturmediumskonzentration des SGK1-Inhibitors aufgetragen und für die Bewertung der intrazellulären Inhibitorwirksamkeit (IC50-Wert) des SGK1-Inhibitors verwendet. (Siehe auch: Exploitation of KESTREL to identify NDRG family members as physiological substrates for SGK1 and GSK3. Murray JT, Campbell DG, Morrice N, Auld GC, Shpiro N, Marquez R, Peggie M, Bain J, Bloomberg GB, Grahammer F, Lang F, Wulff P, Kuhl D, Cohen P.; Biochem. J., 2004 Dez 15; 384 (Pt 3):477-88).

Vor- und nachstehend sind alle Temperaturen in °C angegeben. Abkürzungen:
MS = Massenspektrometrie
DAPECI (WSC) = N-(3-Dimethylaminopropyl)-N'-ethylcarbodiimidhydrochlorid DMF = Dimethylformamid

### Beispiel 1

### 2-Ethyl-4-hydroxy-3-methyl-benzoesäure-N'-[2-(3,4-difluor-5-methoxy-phenyl)-acetyl]-hydrazid (1)

Die Synthese erfolgt analog nachstehenden Schemata:

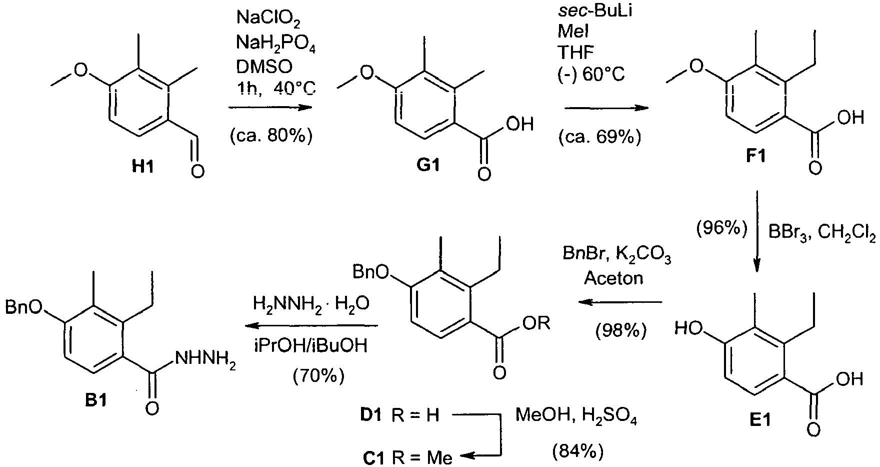

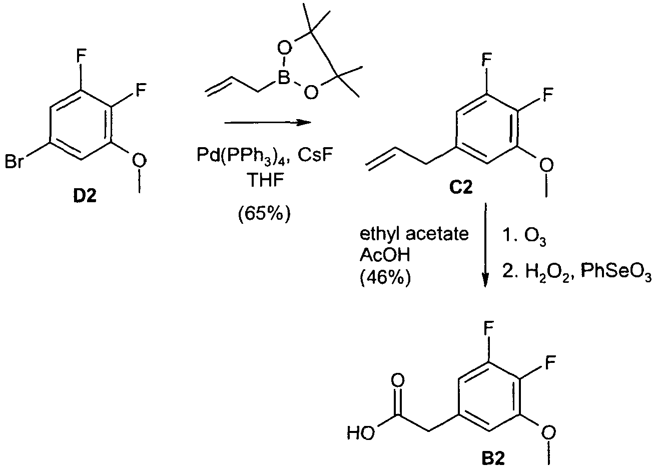

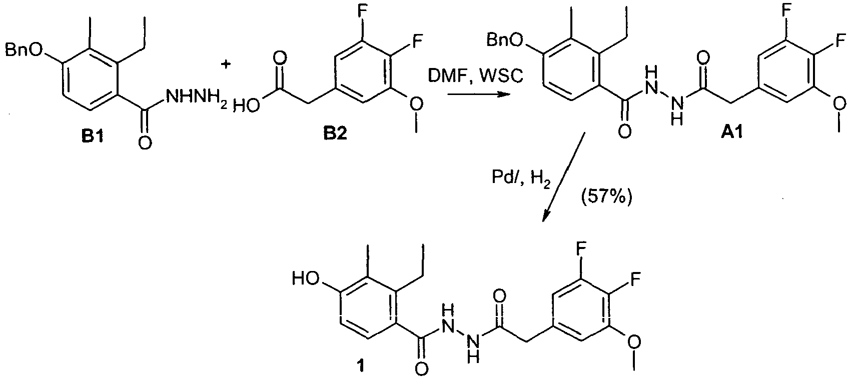

320 mg 4-Benzyloxy-2-ethyl-3-methyl-benzoesäure-*N*'-[2-(3,4-difluor-5-methoxy-phenyl)-acetyl]-hydrazid (A1) werden in 160 ml Methanol gelöst und am H-Cube® (Fa. ThalesNano) an 10% Pd/C (30 x 4 mm Kartusche) hydriert (Fluss: 1 ml/min, Modus: *full H₂,* 30°C, Normaldruck). Anschließend wird die Reaktionslösung zur Trockne eingeengt und mittels Flashsäulenchromatographie an Kieselgel (Lösemittelgradient: Dichlormethan / 0-10 vol.% Methanol) aufgereinigt. 148 mg der Titelverbindung werden durch Gefriertrocknung aus Acetonitril als amorphes, farbloses Lyophilisat erhalten; MS: 378,7 (MH⁺), 779,2 (2M+Na⁺); DC: R_{f}= 0,50 (Kieselgel 60 F254 HPTLC, Dichlormethan/Methanol 95:5 Volumenanteile), Fp. 217°C; ¹H NMR (500,13 MHz, DMSO-d₆): δ [ppm] 10,05, 9,80, 9,56 (3 s, 3 H, OH, 2 NH), 7,06 (dt, 1 H, ⁴*J*_{(H,F)} = 7,2 Hz, ⁵*J*_{(H,F)} = 2,0 Hz, ⁴*J*_{(2',6')} = 2,0 Hz, 6'-H), 7,04 (d, 1 H, ³*J*(_{5,6)} = 8,2 Hz, 6-H), 6,95 (ddd, 1 H, ³*j*_{(H,F)} = 11,1 Hz, ⁴*J*_{(H,F)} = 6,7 Hz, ⁴*J*_{(2',6')} = 2,0 Hz, 2'-H), 6,66 (d, 1 H, ³*J*_{(5,6)} = 8,2 Hz , 5-H), 3,88 (s, 3 H, OCH₃), 3,51 (s, 2 H, CH₂), 2,70 (q, 2 H, ³*J*_{(CH2, CH3)} = 7,5 Hz, CH₂ [Et]), 2,10 (s, 3 H, CH₃), 1,07 (t, 3 H, ³*J*_{(CH3, CH2)} = 7,5 Hz, CH₃ [Et]).

### Beispiel 2

### 2-Ethyl-4-hydroxy-3-methyl-benzoesäure-N'-[2-(3,4-difluor-6-methoxy-phenyl)-acetyl]-hydrazid (2)

Die Synthese erfolgt analog nachstehendem Schema:

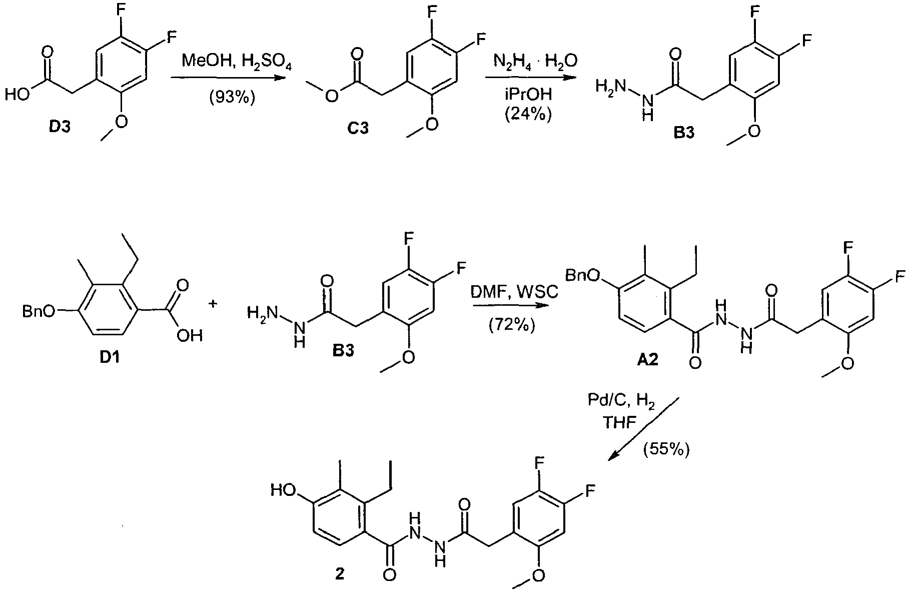

110 mg 4-Benzyloxy-2-ethyl-3-methyl-benzoesäure-*N*'-[2-(3,4-difluor-6-methoxy-phenyl)-acetyl]-hydrazid (A2) werden in 10 ml Tetrahydrofuran gelöst. Anschließend werden 220 mg 5%Pd/C zugegeben. Die Reaktionssuspension wird unter Normalbedingungen 18 h in einer Wasserstoffatmosphäre turbulent gerührt, anschließend über Kieselguhr abgesaugt und das dabei erhaltene Filtrat zur Trockne eingeengt. Der Rückstand wird aus einem Lösemittelgemisch bestehend aus 2-Propanol/Cyclohexan ausgefällt. Der farblose Feststoff wird abgetrennt und im Vakuum bei 70°C 2 h getrocknet, wobei 49 mg der Titelverbindung mit einem Schmelzpunkt von 220,7°C isoliert werden; MS: 378,27 (M⁺); DC: R_{f} = 0,63 (Methyl-*tert-*butylether);
¹H NMR (400,4 MHz, DMSO-d₆): δ [ppm] 9,93, 9,76, 9,57 (3 s, 3 H, OH, 2 NH), 7,39 (dd, 1 H, ³*J*_{(H,F)} = 11,0 Hz, ⁴*J*_{(H,F)} = 9,7 Hz, 2'-H), 7,11 (dd, 1 H, ³*J*_{(H,F)} = 12,8 Hz, ⁴*J*_{(H,F)} = 7,0 Hz, 5'-H), 7,03 (d, 1 H, ³*J*_{(6,5)} = 8,2 Hz, 6-H), 6,65 (d, 1 H, ³*J*_{(5,6)} = 8,2 Hz, 5-H), 3,77 (s, 3 H, OCH₃), 3,46 (s, 2 H, CH₂), 2,70 (q, 2 H, ³*J*_{(CH2, CH3)} = 7,5 Hz, CH₂ [Et]), 2,10 (s, 3 H, CH₃), 1,07 (t, 3 H, ³*J*_{(CH3, CH2)} = 7,5 Hz, CH₃ [Et]).

### Beispiel 3

### 2-Ethyl-4-hydroxy-3-methyl-benzoesäure-N'-[2-(3,4-difluor-phenyl)-acetyl]-hydrazid (3)

Die Synthese erfolgt analog nachstehendem Schema:

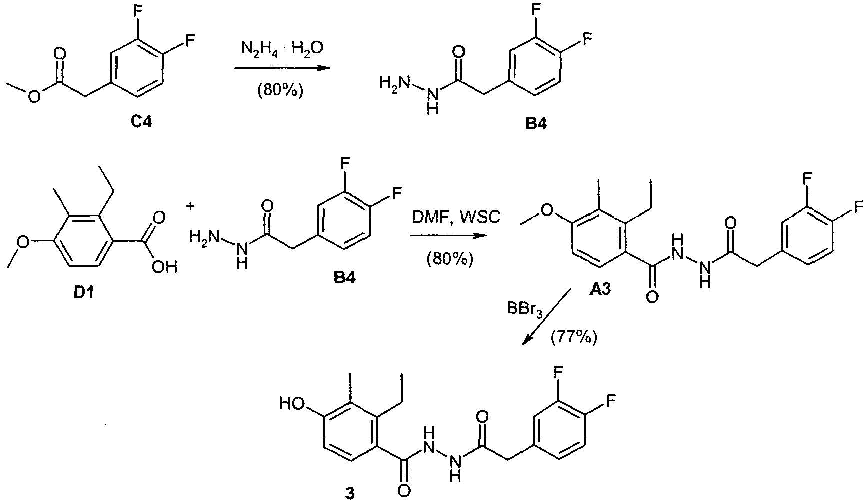

270 mg 2-Ethyl-4-methoxy-3-methyl-benzoesäure-*N*'-[2-(3,4-difluorphenyl)-acetyl]-hydrazid (A3) werden in 3.0 ml getrocknetem Dichlormethan unter einer Stickstoffatmosphäre suspendiert. Anschließend wird 1.0 ml Bortribromid bei Raumtemperatur zugetropft. Danach wird die gelborange Reaktionslösung 18 h über Nacht gerührt. Nach Beendigung der Reaktion wird auf Eis gegossen und anschließend zweimal mit Essigsäureethylester (je 50 ml) extrahiert. Die organischen Phasen werden vereint und einmal mit Wasser gewaschen, anschließend über Na₂SO₄ getrocknet, abgesaugt und das erhaltene Filtrat im Vakuum zur Trockne eingeengt. Der Rückstand wird aus Essigsäureethylester ausgefällt. Der farblose, amorphe Feststoff wird abgetrennt und im Vakuum getrocknet, wobei 217 mg der Titelverbindung mit einem Schmelzpunkt von 242°C isoliert werden; MS: 719,2 (2M+Na⁺); DC: R_{f} = 0,37 (Dichlormethan/Ethanol 10:1 Volumenanteile);
¹H NMR (400,13 MHz, DMSO-d₆): δ [ppm] 10,09, 9,81, 9,61 (3 s, 3 H, OH, 2 NH), 7,42 - 7,34 (m, 2 H, 2'-H, 5'-H), 7,16 (m, 1 H, 6'-H), 7,04 (d, 1 H, ³*J*_{(5,6)} = 8,2 Hz, 6-H), 6,66 (d, 1 H, ³*J*₍₅,₆₎ = 8,2 Hz, 5-H), 3,53 (s, 2 H, CH₂), 2,69 (q, 2 H, ³*J*_{(CH2, CH3)} = 7,5 Hz, CH₂ [Et]), 2,10 (s, 3 H, CH₃), 1,07 (t, 3 H, ³*J*_{(CH3, CH2)} = 7,5 Hz, CH₃ [Et]).

### Herstellung der Zwischenverbindungen

### 4-Benzyloxy-2-ethyl-3-methyl-benzoesäure-N'-[2-(3,4-difluor-5-methoxy-phenyl)-acetyl]-hydrazid (A1)

340 mg 2-(3,4-Difluor-5-methoxy-phenyl)-essigsäure (B2) werden in 7,0 ml N,N-Dimethylformamid unter einer trockenen Argonatmosphäre gelöst. Anschließend werden 484,3 mg N-(3-Dimethylaminopropyl)-N'-ethylcarbo-diimidhydrochlorid, 130,2 mg N-Hydroxybenzotriazol und 526,0 mg 4-Benzyloxy-2-ethyl-3-methyl-benzoesäure-hydrazid (B1) zugegeben. Die Reaktionslösung wird 18 h über Nacht bei Raumtemperatur gerührt. Nach Beendigung der Reaktion (DC-Kontrolle) wird mit 100 ml Wasser versetzt, 30 min gerührt und anschließend dreimal mit je 75 ml Essigsäureeethylester extrahiert. Der vereinigten organischen Phasen werden über Na₂SO₄ getrocknet, abgesaugt und das erhaltene Filtrat im Vakuum zur Trockne eingeengt. Der Rückstand wird mittels Flashsäulenchromatographie an Kieselgel (Lösemittelgradient: Dichlormethan / 0-20 vol.% Ethanol) aufgereinigt, wobei 321 mg der Titelverbindung als farbloses Öl erhalten werden; MS: 937,3 (2M+H⁺); DC: R_{f} = 0,50 (Dichlormethan/Methanol 95:5 Volumenanteile).

### 4-Benzyloxy-2-ethyl-3-methyl-benzoesäure-N'-[2-(3,4-difluor-6-methoxy-phenyl)-acetyl]-hydrazid (A2)

113 mg 4-Benzyloxy-2-ethyl-3-methyl-benzoesäure (D1), 90 mg 2-(3,4-Difluor-6-methoxy-phenyl)-acetyl-hydrazid (B3), 120 mg N-(3-Dimethyl-aminopropyl)-N'-ethylcarbodiimidhydrochlorid und 32 mg N-Hydroxybenzotriazol werden in 2,0 ml N,N-Dimethylformamid unter einer trockenen Argonatmosphäre gelöst. Die Reaktionslösung wird 18 h über Nacht bei Raumtemperatur gerührt. Nach Beendigung der Reaktion (DC-Kontrolle) wird die klare Lösung mit 30 ml Wasser verdünnt und 30 min gerührt. Anschließend wird der entstandene Niederschlag abgesaugt und mit kaltem Wasser mehrfach nachgewaschen. Der Filterkuchen wird danach aus 2-Propanol umkristallisiert. Der Niederschlag wird abgesaugt und im Vakuum bei 70°C getrocknet, wobei 140 mg der Titelverbindung als farbloser Feststoff mit einem Schmelzpunkt von 224°C isoliert werden; MS: 468 (M⁺); DC: R_{f} = 0.50 (Cyclohexan/Methyl-*tert*-butylether 1:4 Volumenanteile).

### 2-Ethyl-4-methoxy-3-methyl-benzoesäure-N'-[2-(3,4-difluor-phenyl)-acetyl]-hydrazid (A3)

194 mg 4-Benzyloxy-2-ethyl-3-methyl-benzoesäure (D1), 186 mg 2-(3,4-Difluorphenyl)-acetyl-hydrazid (B4), 288 mg N-(3-Dimethylaminopropyl)-N'-ethylcarbodiimidhydrochlorid und 77 mg N-Hydroxybenzotriazol werden in 3,0 ml N,N-Dimethylformamid unter einer trockenen Argonatmosphäre gelöst. Die Reaktionslösung wird 18 h über Nacht bei Raumtemperatur gerührt. Nach Beendigung der Reaktion (DC-Kontrolle) wird die klare Lösung mit 50 ml Wasser verdünnt und 30 min gerührt. Anschließend wird der entstandene Niederschlag abgesaugt und mit kaltem Wasser mehrfach nachgewaschen, abgesaugt und im Vakuum bei 70°C getrocknet, wobei 290 mg der Titelverbindung als farbloser Feststoff mit einem Schmelzpunkt von 225°C isoliert werden; MS: 747,2 (2M+Na⁺); DC: R_{f} = 0.62 (Methyl-*tert*-butylether).

### 4-Benzyloxy-2-ethyl-3-methyl-benzoesäure-hydrazid (B1)

17,5 g 4-Benzyloxy-2-ethyl-3-methyl-benzoesäuremethylester (C1) werden mit 20 ml 2-Propanol und 10 ml Isobutanol (2-Methylpropan-1-ol) sowie 20,0 ml Hydraziniumhydroxid versetzt. Die Reaktionsmischung wird anschließend 18 h über Nacht am Rückfluss erhitzt. Nach dem Abkühlen werden der Lösung weitere 30 mi 2-Propanol zugesetzt, die anschließen 30 min bei Raumtemperatur gerührt wird. Der Niederschlag wird abgesaugt und der Filterkuchen mit wenig 2-Propanol mehrfach nachgewaschen. Anschließend wird aus 2-Propanol umkristallisiert und der erhaltene Niederschlag im Vakuum über Nacht getrocknet, wobei 12,3 g der Titelverbindung als farbloser, amorpher Feststoff mit einem Schmelzpunkt von 179°C isoliert werden; MS: 284,2 (M⁺); DC: R_{f} = 0,27 (Essigsäureethylester/Ethanol 97:3 Volumenanteile).

### 2-(3,4-Difluor-5-methoxy-phenyl)-essigsäure (B2)

1,59 g 5-Allyl-1,2-difluor-3-methoxy-benzol (C2) werden in 5,25 ml Essigsäureethylester und 19,75 ml Eisessig gelöst und auf 0°C im Eisbad gekühlt. Die Lösung wird anschließend 15 min mit Ozon behandelt (Ozongenerator: Sauerstofffluss 40 I/h- entspricht 5 g/h O₃). Anschließend wird mit 12 ml Wasser verdünnt und für weitere 15 min auf 50°C erwärmt. Danach wird die Reaktionslösung im Vakuum eingeengt und der verbleibende Rückstand in 50 ml Tetrahydrofuran gelöst. Es werden 420 µl H₂O₂ (30%ige Lösung) sowie 163 mg Phenylselensäure zugefügt. Anschließend wird die Reaktionslösung 2 h am Rückfluß erhitzt und nach Beendigung der Reaktion im Vakuum zur Trockne eingeengt. Der Rückstand wird mittels Flashsäulenchromatographie an Kieselgel (Lösemittelgradient: Dichlormethan / 0-40vol.% Methanol) aufgereinigt, wobei 806 mg der Titelverbindung als farbloses Öl erhalten werden; MS: 203,1 (MH⁺); DC: R_{f}= 0,40 (Cyclohexan/Essigsäureethylester 9:1).

### 2-(3,4-Difluor-6-methoxy-phenyl)-acetyl-hydrazid (B3)

2,5 g 2-(3,4-Difluor-6-methoxy-phenyl)-essigsäuremethylester (C3) werden mit 35 ml 2-Propanol und 618 µl Hydraziniumhydroxid versetzt. Die Reaktionsmischung wird anschließend 18 h über Nacht am Rückfluss erhitzt. Anschließend wird im Vakuum zur Trockne eingeengt und mittels Flashsäulenchromatographie an Kieselgel (Lösemittelgradient: Essigsäureethylester i 0-20vol.% Ethanol) aufgereinigt, wobei 590 mg der Titelverbindung als farbloser Feststoff mit einem Schmelzpunkt von 139,7°C isoliert werden; MS: 216,1 (M⁺); DC: R_{f}= 0,30 (Essigsäureethylester/Ethanol 9:1 Volumenanteile).

### 2-(3,4-Difluorphenyl)-acetyl-hydrazid (B4)

### (analog WO2004/101512A2, Bioorg. Med. Chem. Lett. 2004, 14(3), 817-822)

1,0 g 2-(3,4-Difluorphenyl)-essigsäuremethylester (C4) wird in 6,0 ml 2-Propanol gelöst. Anschließend werden 365 µl Hydraziniumhydroxid zugegeben und die Reaktionslösung 18 h über Nacht am Rückfluss erhitzt. Danach wird im Vakuum zur Trockne eingeengt und mittels Flashsäulenchromatographie an Kieselgel (Lösemittelgradient: Essigsäureethylester / 0-10vol.% Ethanol) aufgereinigt, wobei 803 mg der Titelverbindung als farbloser Feststoff mit einem Schmelzpunkt von 112°C isoliert werden; MS: 187,1 (MH⁺); DC: R_{f}= 0,50 (Essigsäureethylester/Ethanol 9:1 Volumenanteile).

### 4-Benzyloxy-2-ethyl-3-methyl-benzoesäuremethylester (C1)

19,7 g 4-Benzyloxy-2-ethyl-3-methyl-benzoesäure (D1) werden in 200 ml Methanol gelöst. Anschließend werden 5,0 ml H₂SO₄ (95-98%, reinst) zugegeben. Die Reaktionslösung wird 18 h über Nacht bei 67°C am Rückfluss erhitzt, anschließend auf 1/3 des Volumens im Vakuum eingeengt und 200 ml Wasser zugesetzt. Danach wird zweimal mit je 200 ml Essigsäureethylester extrahiert. Die vereinigten organischen Phasen werden anschließend mit gesättigter NaHCO₃-Lösung gewaschen, über Na₂SO₄ getrocknet und im Vakuum eingeengt, wobei 17,5 g der Titelverbindung als farbloses Öl isoliert werden; MS: 285,2 (MH⁺); DC: R_{f} = 0,72 (Cyclohexan/Methyl-*tert*-butylether 1:1 Volumenanteile).

### 5-Allyl-1, 2-difluor-3-methoxy-benzol (C2)

3,06 g 1-Brom-3,4-difluor-5-methoxy-benzol (D2), 4,54 ml Allylboronsäurepinacolester, 3,04 g Tetrakis(triphenylphosphan)-palladium(0) und 7,62 g Cäsiumfluorid werden in 115 ml Tetrahydrofuran unter einer Argonatmosphäre suspendiert. Anschließend wird die Reaktionsmischung 48 h am Rückfluss erhitzt. Zur Aufarbeitung wird mit 400 ml Diethylether verdünnt und mit je 100 ml Wasser und gesättigter NaCl-Lösung extrahiert. Die vereinigten organischen Phasen werden über Na₂SO₄ getrocknet, filtriert und im Vakuum zur Trockne eingeengt. Der Rückstand wird mittels Flashsäulenchromatographie an Kieselgel (Lösemittel: Cyclohexan) aufgereinigt, wobei 1,59 g der Titelverbindung als farbloses Öl erhalten werden; MS: 184,0 (M⁺); DC: R_{f} = 0,69 (Cyclohexan/Essigsäureethylester 8:1 Volumenanteile).

### 2-(3,4-Difluor-6-methoxy-phenyl)-essigsäuremethylester (C3)

4,04 g 2-(3,4-Difluor-6-methoxy-phenyl)-essigsäure (D3) werden in 34 ml Methanol gelöst. Anschließend werden 1,54 ml H₂SO₄ (95-98%, reinst) zugefügt und die Reaktionslösung 3 h am Rückfluss erhitzt. Zur Aufarbeitung wird mit 100 ml Wasser verdünnt und zweimal mit je 150 ml Essigsäureethylester extrahiert. Die vereinigten organischen Phasen werden mit 50 ml gesättigter NaHCO₃-Lösung gewaschen, über Na₂SO₄ getrocknet, abgesaugt und im Vakuum zur Trockne eingeengt. Der Rückstand wird aus Cyclohexan umkristallisiert, wobei 4,0 g der Titelverbindung als farbloser Feststoff mit einem Schmelzpunkt von 48,3°C isoliert werden; MS: 216,1 (M⁺); DC: R_{f}= 0,60 (Cyclohexan/Diethylether 1:1 Volumenanteile).

### 2-(3,4-Difluorphenyl)-essigsäuremethylester (C4)

Verbindung C4 ist kommerziell erhältlich.

### 4-Benzyloxy-2-ethyl-3-methyl-benzoesäure (D1)

2,10 g 2-Ethyl-4-hydroxy-3-methyl-benzoesäure (E1) werden in 50 ml Aceton gelöst. Anschließend werden 3,30 ml Benzylbromid und 5,10 g K₂CO₃ zugefügt. Die Reaktionssuspension wird 18 h über Nacht am Rückfluss erhitzt, anschließend abgesaugt und das erhaltene Filtrat im Vakuum zur Trockne eingeengt. Der Rückstand wird in 40 ml Ethanol gelöst und mit 40 ml 2,0 N NaOH-Lösung versetzt. Danach wird 3 h am Rückfluss erhitzt. Die klare Lösung wird mit 150 ml Wasser verdünnt und mit 20%iger Salzsäure auf pH1 eingestellt. Nach 30 min rühren wird der entstandene Niederschlag abgesaugt, mit Wasser nachgewaschen und bei 90°C im Vakuum über Nacht getrocknet, wobei 2,45 g der Titelverbindung als beiger, amorpher Feststoff mit einem Schmelzpunkt von 169°C isoliert werden; MS: 270,0 (M⁺); DC: R_{f} = 0,36 (Cyclohexan/Essigsäureeethylester 2:1 Volumenanteile).

### 1-Brom-3,4-difluor-5-methoxy-benzol (D2)

Verbindung D2 ist kommerziell erhältlich.

### 2-(3,4-Difluor-6-methoxy-phenyl)-essigsäure (D3)

Verbindung D2 ist kommerziell erhältlich.

### 2-Ethyl-4-hydroxy-3-methyl-benzoesäure (E1)

10,0 g 2-Ethyl-4-methoxy-3-methyl-benzoesäure (F1) werden in 50 ml Dichlormethan unter einer Stickstoffatmosphäre suspendiert. Anschließend werden unter Eisbadkühlung 29,3 ml Bortribromid langsam zugetropft. Die erhaltene transparente, rote Lösung wird bei Raumtemperatur 1 h gerührt. Anschließend wird vorsichtig unter Rühren auf 600 ml Eiswasser gegossen. Die wässrige Phase wird 30 min gerührt und anschließend zweimal mit je 200 ml Essigsäureethylester extrahiert. Die vereinigten organischen Phasen werden einmal mit 200 ml Wasser gewaschen, über Na₂SO₄ getrocknet, im Vakuum eingeengt und mittels Flashsäulenchromatographie an Kieselgel (Lösemittelgradient: Cyclohexan / 0-100vol.% Essigsäureethylester) aufgereinigt, wobei 9,37 g der Titelverbindung als farbloser, amorpher Feststoff mit einem Schmelzpunkt von 139°C isoliert werden; MS: 180,0 (M⁺); DC: R_{f} = 0,33 (Cyclohexan/Methyl-*tert*-butylether 3:2 Volumenanteile).

### 2-Ethyl-4-methoxy-3-methyl-benzoesäure (F1)

2,50 g 4-Methoxy-2,3-dimethyl-benzoesäure (G1) werden in 160 ml Tetrahydrofuran unter einer Stickstoffatmosphäre gelöst und auf (-) 78°C gekühlt. Anschließend werden 11,5 ml sec-BuLi so zugetropft, dass die Temperatur der Reaktionslösung (-) 65°C nicht übersteigt. Nach Beendigung der Zugabe wird die rötliche Reaktionslösung weitere 30 min unter Kühlung gerührt. Danach werden langsam 3,59 ml Methyliodid zugetropft. Anschließend wird die Kühlung entfernt und 1 h gerührt. Zum Aufarbeiten werden vorsichtig 160 ml Wasser zugefügt. Die Lösung wird anschließend zweimal mit je 130 ml Essigsäureethylester extrahiert. Die wässrige Phase wird im Eisbad unter Rühren gekühlt, mit 1.0 M HCl angesäuert und nach 30 min filtriert. Der Filterkuchen wird mit kaltem Wasser nachgewaschen und anschließend in 2-Propanol umkristallisiert. Das Kristallisat wird abgesaugt und im Vakuum bei 70°C 2 h getrocknet, wobei 1,90 g der Titelverbindung als farbloser, kristalliner Feststoff mit einem Schmelzpunkt von 176,7°C isoliert werden; MS: 194,2 (M⁺); DC: R_{f} = 0,29 (Diethylether/Petrolether 1:1 Volumenanteile).

### 4-Methoxy-2,3-dimethyl-benzoesäure (G1)

Verbindung G1 kann nach EP1666473 A1, S. 41 aus kommerziell erhältlichem 4-Methoxy-2,3-dimethyl-benzaldehyd (H1) hergestellt werden.

### Pharmakologische Daten

**Tabelle 1 SGK1-Inhibierung**

| Verbindung Nr. | Inhibierung IC₅₀ (Enzym) | Inhibierung IC₅₀ (Zelle) |
|---|---|---|
| 1 | < 10 nM | < 500 nM |
| 2 | > 10 nM | > 500 nM |
| 3 | > 10 nM | > 500 nM |

Die nachfolgenden Beispiele betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄ 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatinekapseln gefüllt, so daß jede Kapsel 20 mg des Wirkstoffs enthält.

### Beispiel H: Ampullen

Eine Lösung von 1 kg eines erfindungsgemäßen Wirkstoffes in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 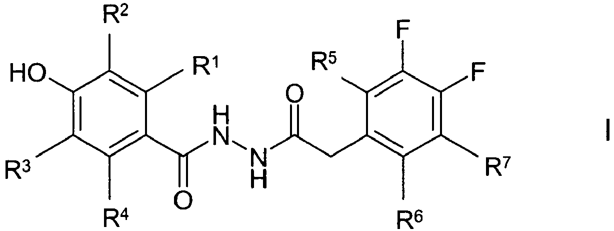 worin
R¹, R⁴, R⁵ jeweils unabhängig voneinander H, Hal, A oder CN,
R², R³ jeweils unabhängig voneinander H, Hal oder A,
R⁶, R⁷ jeweils unabhängig voneinander H, A, OA, NHA oder NA₂,
A Alkyl mit 1-6 C-Atomen, worin 1-5 H-Atome durch F ersetzt sein können oder Cycloalkyl mit 3-7 C-Atomen,
Hal F, Cl, Br oder I,
bedeuten,
sowie ihre pharmazeutisch verwendbaren Salze und Stereoisomere, einschließlich deren Mischungen in allen Verhältnissen.

2. Verbindungen nach Anspruch 1, worin
R¹, R² A bedeuten,
sowie ihre pharmazeutisch verwendbaren Salze und Stereoisomere, einschließlich deren Mischungen in allen Verhältnissen.

3. Verbindungen nach Anspruch 1 oder 2, worin
R³, R⁴ H bedeuten,
sowie ihre pharmazeutisch verwendbaren Salze und Stereoisomere, einschließlich deren Mischungen in allen Verhältnissen.

4. Verbindungen nach Anspruch 1, 2 oder 3, worin
R⁵ H bedeutet,
sowie ihre pharmazeutisch verwendbaren Salze und Stereoisomere, einschließlich deren Mischungen in allen Verhältnissen.

5. Verbindungen nach nach einem oder mehreren der Ansprüche 1-4, worin
R⁶, R⁷ jeweils unabhängig voneinander H oder OA bedeuten, sowie ihre pharmazeutisch verwendbaren Salze und Stereoisomere, einschließlich deren Mischungen in allen Verhältnissen.

6. Verbindungen nach einem oder mehreren der Ansprüche 1-5, worin A Methyl, Ethyl, Propyl oder Isopropyl,
bedeutet,
sowie ihre pharmazeutisch verwendbaren Salze und Stereoisomere, einschließlich deren Mischungen in allen Verhältnissen.

7. Verbindungen nach einem oder mehreren der Ansprüche 1-6, worin
R¹, R² A,
R³, R⁴ H,
R⁵ H,
R⁶, R⁷ jeweils unabhängig voneinander H oder OA,
A Alkyl mit 1-6 C-Atomen, worin 1-5 H-Atome durch F ersetzt sein können,
Hal F, Cl, Br oder I,
bedeuten,
sowie ihre pharmazeutisch verwendbaren Salze und Stereoisomere, einschließlich deren Mischungen in allen Verhältnissen.

8. Verbindungen nach Anspruch 1 ausgewählt aus der Gruppe
| Nr. | Strukturformel und/oder Name |
|---|---|
| 1 | 2-Ethyl-4-hydroxy-3-methyl-benzoesäure-N'-[2-(3,4-difluor-5-methoxy-phenyl)-acetyl]-hydrazid |
| 2 | 2-Ethyl-4-hydroxy-3-methyl-benzoesäure-N'-[2-(3,4-difluor-6-methoxy-phenyl)-acetyl]-hydrazid |
| 3 | 2-Ethyl-4-hydroxy-3-methyl-benzoesäure-N'-[2-(3,4-difluor-phenyl)-acetyl]-hydrazid |
sowie ihre pharmazeutisch verwendbaren Salze, einschließlich deren Mischungen in allen Verhältnissen.

9. Verfahren zur Herstellung von Verbindungen der Formel I nach den Ansprüchen 1-8 sowie ihrer pharmazeutisch verwendbaren Salze und Stereoisomere, **dadurch gekennzeichnet, daß** man
a) eine Verbindung der Formel II 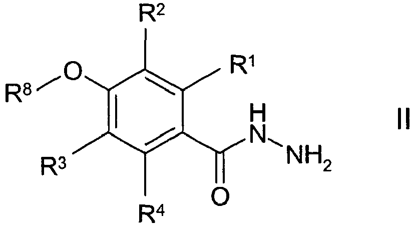 worin
R¹, R², R³, R⁴ die in Anspruch 1 angegebenen Bedeutungen haben, und R⁸ eine Hydroxyschutzgruppe bedeutet,
mit einer Verbindung der Formel III 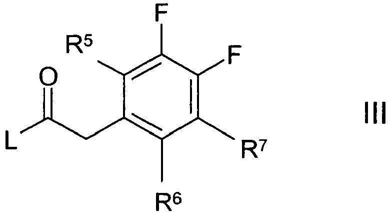 worin
L Cl, Br, I oder eine freie oder reaktionsfähig funktionell abgewandelte OH-Gruppe bedeutet und
R⁵, R⁶ und R⁷ die in Anspruch 1 angegebenen Bedeutungen haben,
umsetzt, und anschließend R⁸ abspaltet
oder
b) eine Verbindung der Formel IV 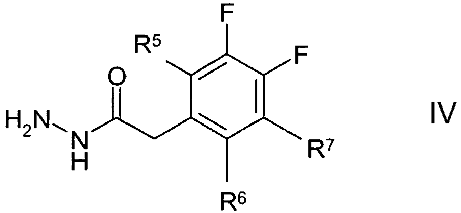 worin
R⁵, R⁶ und R⁷ die in Anspruch 1 angegebenen Bedeutungen haben,
mit einer Verbindung der Formel V 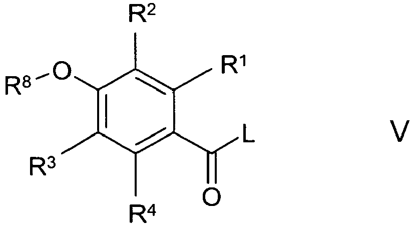 worin
L Cl, Br, I oder eine freie oder reaktionsfähig funktionell abgewandelte OH-Gruppe bedeutet,
R¹, R², R³ und R⁴ die in Anspruch 1 angegebenen Bedeutungen haben, und R⁸ eine Hydroxyschutzgruppe bedeutet,
umsetzt, und anschließend R⁸ abspaltet
oder
c) sie aus einem ihrer funktionellen Derivate durch Behandeln mit einem solvolysierenden oder hydrogenolysierenden Mittel in Freiheit setzt,
und/oder eine Base oder Säure der Formel I in eines ihrer Salze umwandelt.

10. Arzneimittel, enthaltend mindestens eine Verbindung nach Anspruch 1-8 und/oder ihre pharmazeutisch verwendbaren Salze und Stereoisomere, einschließlich deren Mischungen in allen Verhältnissen, sowie gegebenenfalls Träger- und/oder Hilfsstoffe.

11. Verwendung von Verbindungen gemäß Anspruch 1-8, sowie ihrer pharmazeutisch verwendbaren Derivate, Solvate und Stereoisomere, einschließlich deren Mischungen in allen Verhältnissen, zur Herstellung eines Arzneimittels zur Behandlung oder Vorbeugung von Diabetes, Fettsucht, metabolischem Syndrom (Dyslipidämie), systemischer und pulmonaler Hypertonie, Herzkreislauferkrankungen und Nierenerkrankungen, allgemein bei jeglicher Art von Fibrosen und entzündlichen Prozessen, Krebs, Tumorzellen, Tumormetastasen, Koagulopathien, neuronaler Erregbarkeit, Glaukom, Katarakt, bakteriellen Infektionen sowie in einer antiinfektiösen Therapie, zur Steigerung der Lernfähigkeit und Aufmerksamkeit, sowie zur Behandlung und Prophylaxe von Zellalterung und Stress und zur Behandlung von Tinitus.

12. Verwendung nach Anspruch 11, wobei es sich bei Diabetes um Diabetes mellitus, diabetische Nephropathie, diabetische Neuropathie, diabetische Angiopathie und Mikroangiopathie handelt.

13. Verwendung nach Anspruch 11, wobei es sich bei Herzkreislauferkrankungen um kardiale Fibrosen nach Myokardinfarkt, Herzhypertrophie, Herzinsuffizienz und Arteriosklerose handelt.

14. Verwendung nach Anspruch 11, wobei es sich bei Nierenerkrankungen um Glomerulosklerose, Nephrosklerose, Nephritis, Nephropathie und Störung der Elektrolytausscheidung handelt.

15. Verwendung nach Anspruch 11, wobei es sich bei Fibrosen und entzündlichen Prozessen um Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung und Morbus Alzheimer handelt.

16. Arzneimittel enthaltend mindestens eine Verbindung gemäß Anspruch 1-8 und/oder ihre pharmazeutisch verwendbaren Salze und Stereoisomere, einschließlich deren Mischungen in allen Verhältnissen, und mindestens einen weiteren Arzneimittelwirkstoff.

17. Set (Kit), bestehend aus getrennten Packungen von
(a) einer wirksamen Menge an einer Verbindung gemäß Anspruch 1-8 und/oder ihrer pharmazeutisch verwendbaren Salze und Stereoisomere, einschließlich deren Mischungen in allen Verhältnissen,
und
(b) einer wirksamen Menge eines weiteren Arzneimittelswirkstoffs.

## Claims

1. Compounds of the formula I 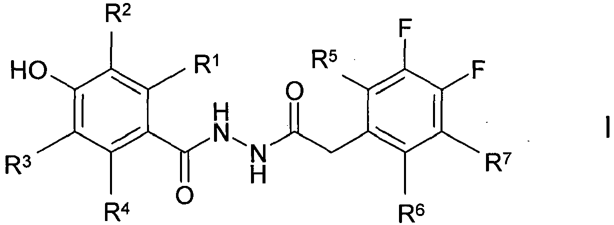 in which
R¹, R⁴, R⁵ each, independently of one another, denote H, Hal, A or CN,
R², R³ each, independently of one another, denote H, Hal or A,
R⁶, R⁷ each, independently of one another, denote H, A, OA, NHA or NA₂,
A denotes alkyl having 1-6 C atoms, in which 1-5 H atoms may be replaced by F, or cycloalkyl having 3-7 C atoms,
Hal denotes F, Cl, Br or I,
and pharmaceutically usable salts and stereoisomers thereof, including mixtures thereof in all ratios.

2. Compounds according to Claim 1 in which
R¹, R² denote A,
and pharmaceutically usable salts and stereoisomers thereof, including mixtures thereof in all ratios.

3. Compounds according to Claim 1 or 2 in which
R³, R⁴ denote H,
and pharmaceutically usable salts and stereoisomers thereof, including mixtures thereof in all ratios.

4. Compounds according to Claim 1, 2 or 3 in which
R⁵ denotes H,
and pharmaceutically usable salts and stereoisomers thereof, including mixtures thereof in all ratios.

5. Compounds according to one or more of Claims 1-4 in which
R⁶, R⁷ each, independently of one another, denote H or OA, and pharmaceutically usable salts and stereoisomers thereof, including mixtures thereof in all ratios.

6. Compounds according to one or more of Claims 1-5 in which A denotes methyl, ethyl, propyl or isopropyl,
and pharmaceutically usable salts and stereoisomers thereof, including mixtures thereof in all ratios.

7. Compounds according to one or more of Claims 1-6 in which
R¹, R² denote A,
R³, R⁴ denote H,
R⁵ denotes H,
R⁶, R⁷ each, independently of one another, denote H or OA,
A denotes alkyl having 1-6 C atoms, in which 1-5 H atoms may be replaced by F,
Hal denotes F, Cl, Br or I,
and pharmaceutically usable salts and stereoisomers thereof, including mixtures thereof in all ratios.

8. Compounds according to Claim 1 selected from the group
| No. | Structural formula and/or name |
|---|---|
| 1 | N'-[2-(3,4-Difluoro-5-methoxyphenyl)acetyl]-2-ethyl-4-hydroxy-3-methylbenzohydrazide |
| 2 | N'-[2-(3,4-Difluoro-6-methoxyphenyl)acetyl]-2-ethyl-4-hydroxy-3-methylbenzohydrazide |
| 3 | N'-[2-(3,4-Difluorophenyl)acetyl]-2-ethyl-4-hydroxy-3-methylbenzohydrazide |
and pharmaceutically usable salts thereof, including mixtures thereof in all ratios.

9. Process for the preparation of compounds of the formula I according to Claims 1-8 and pharmaceutically usable salts and stereoisomers thereof, **characterised in that**
a) a compound of the formula II 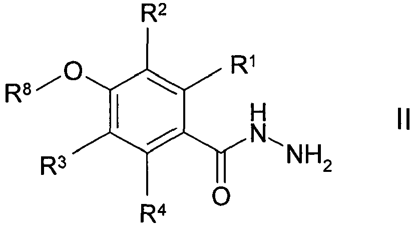 in which
R¹, R², R³, R⁴ have the meanings indicated in Claim 1, and R⁸ denotes a hydroxyl-protecting group,
is reacted with a compound of the formula III 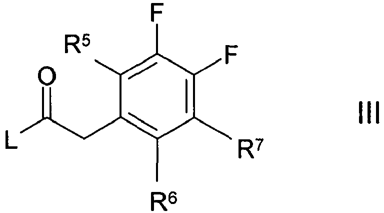 in which
L denotes Cl, Br, I or a free or reactively functionally modified OH group and
R⁵, R⁶ and R⁷ have the meanings indicated in Claim 1,
and R⁸ is subsequently cleaved off,
or
b) a compound of the formula IV 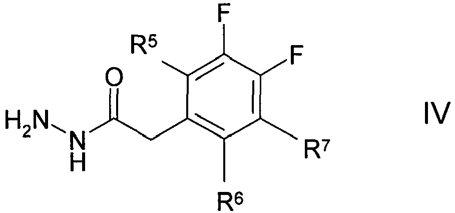 in which
R⁵, R⁶ and R⁷ have the meanings indicated in Claim 1,
is reacted with a compound of the formula V 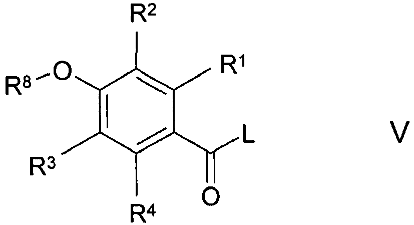 in which
L denotes Cl, Br, I or a free or reactively functionally modified OH group,
R¹, R², R³ and R⁴ have the meanings indicated in Claim 1, and
R⁸ denotes a hydroxyl-protecting group,
and R⁸ is subsequently cleaved off,
or
c) they are liberated from one of their functional derivatives by treatment with a solvolysing or hydrogenolysing agent,
and/or a base or acid of the formula I is converted into one of its salts.

10. Medicaments comprising at least one compound according to Claims 1-8 and/or pharmaceutically usable salts and stereoisomers thereof, including mixtures thereof in all ratios, and optionally excipients and/or adjuvants.

11. Use of compounds according to Claims 1-8, and pharmaceutically usable derivatives, solvates and stereoisomers thereof, including mixtures thereof in all ratios, for the preparation of a medicament for the treatment or prevention of diabetes, obesity, metabolic syndrome (dyslipidaemia), systemic and pulmonary hypertonia, cardiovascular diseases and kidney diseases, generally in any type of fibroses and inflammatory processes, cancer, tumour cells, tumour metastases, coagulopathies, neuronal excitability, glaucoma, cataract, bacterial infections and in anti-infection therapy, for increasing learning ability and attention, and for the treatment and prophylaxis of cell ageing and stress and for the treatment of tinnitus.

12. Use according to Claim 11, where diabetes is diabetes mellitus, diabetic nephropathy, diabetic neuropathy, diabetic angiopathy and microangiopathy.

13. Use according to Claim 11, where cardiovascular diseases are cardiac fibroses after myocardial infarction, cardiac hypertrophy, cardiac insufficiency and arteriosclerosis.

14. Use according to Claim 11, where kidney diseases are glomerulosclerosis, nephrosclerosis, nephritis, nephropathy and electrolyte excretion disorder.

15. Use according to Claim 11, where fibroses and inflammatory processes are liver cirrhosis, pulmonary fibrosis, fibrosing pancreatitis, rheumatism and arthroses, Crohn's disease, chronic bronchitis, radiation fibrosis, sclerodermatitis, cystic fibrosis, scarring and Alzheimer's disease.

16. Medicaments comprising at least one compound according to Claims 1-8 and/or pharmaceutically usable salts and stereoisomers thereof, including mixtures thereof in all ratios, and at least one further medicament active compound.

17. Set (kit) consisting of separate packs of
(a) an effective amount of a compound according to Claims 1-8 and/or pharmaceutically usable salts and stereoisomers thereof, including mixtures thereof in all ratios,
and
(b) an effective amount of a further medicament active compound.

## Revendications

1. Composés de formule I 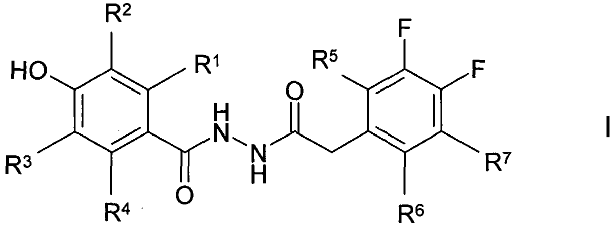 dans lesquels
R¹, R⁴, R⁵ désignent chacun, indépendamment les uns des autres, H, Hal, A ou CN,
R², R³ désignent chacun, indépendamment l'un de l'autre, H, Hal ou A,
R⁶, R⁷ désignent chacun, indépendamment l'un de l'autre, H, A, OA, NHA ou NA₂,
A désigne alkyle ayant 1-6 atomes de C, où 1-5 atomes de H peuvent être remplacés par F, ou
cycloalkyle ayant 3-7 atomes de C,
Hal désigne F, Cl, Br ou I,
ainsi que les sels et stéréoisomères pharmaceutiquement utilisables de ceux-ci, y compris des mélanges de ceux-ci en toutes proportions.

2. Composés selon la revendication 1, dans lesquels
R¹, R² désignent A,
ainsi que les sels et stéréoisomères pharmaceutiquement utilisables de ceux-ci, y compris des mélanges de ceux-ci en toutes proportions.

3. Composés selon la revendication 1 ou 2, dans lesquels
R³, R⁴ désignent H,
ainsi que les sels et stéréoisomères pharmaceutiquement utilisables de ceux-ci, y compris des mélanges de ceux-ci en toutes proportions.

4. Composés selon la revendication 1, 2 ou 3, dans lesquels
R⁵ désigne H,
ainsi que les sels et stéréoisomères pharmaceutiquement utilisables de ceux-ci, y compris des mélanges de ceux-ci en toutes proportions.

5. Composés selon l'une ou plusieurs parmi les revendications 1-4, dans lesquels
R⁶, R⁷ désignent chacun, indépendamment l'un de l'autre, H ou
OA,
ainsi que les sels et stéréoisomères pharmaceutiquement utilisables de ceux-ci, y compris des mélanges de ceux-ci en toutes proportions.

6. Composés selon l'une ou plusieurs parmi les revendications 1-5, dans lesquels
A désigne méthyle, éthyle, propyle ou isopropyle,
ainsi que les sels et stéréoisomères pharmaceutiquement utilisables de ceux-ci, y compris des mélanges de ceux-ci en toutes proportions.

7. Composés selon l'une ou plusieurs parmi les revendications 1-6, dans lesquels
R¹, R² désignent A,
R³, R⁴ désignent H,
R⁵ désigne H,
R⁶, R⁷ désignent chacun, indépendamment l'un de l'autre, H ou OA,
A désigne alkyle ayant 1-6 atomes de C, où 1-5 atomes de H peuvent être remplacés par F,
Hal désigne F, Cl, Br ou I,
ainsi que les sels et stéréoisomères pharmaceutiquement utilisables de ceux-ci, y compris des mélanges de ceux-ci en toutes proportions.

8. Composés selon la revendication 1, choisis dans le groupe constitué par
| n° | Formule structurelle et/ou nom |
|---|---|
| 1 | N'-[2-(3,4-Difluoro-5-méthoxyphényl)acétyl]-2-éthyl-4-hydroxy-3-méthylbenzohydrazide |
| 2 | N'-[2-(3,4-Difluoro-6-méthoxyphényl)acétyl]-2-éthyl-4-hydroxy-3-méthylbenzohydrazide |
| 3 | N'-[2-(3,4-Difluorophényl)acétyl]-2-éthyl-4-hydroxy-3-méthylbenzohydrazide |
ainsi que les sels pharmaceutiquement utilisables de ceux-ci, y compris des mélanges de ceux-ci en toutes proportions.

9. Procédé de préparation de composés de formule I selon les revendications 1-8 et de sels et stéréoisomères pharmaceutiquement utilisables de ceux-ci, **caractérisé en ce que**
a) un composé de formule II 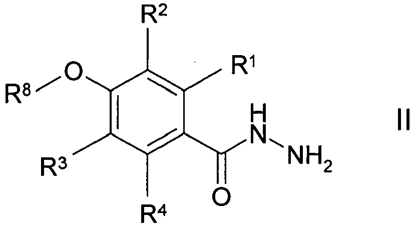 dans lequel
R¹, R², R³, R⁴ revêtent les significations indiquées selon la revendication 1, et
R⁸ désigne un groupement protecteur d'hydroxyle,
est réagi avec un composé de formule III 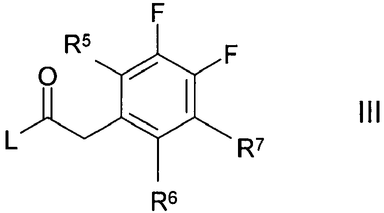 dans lequel
L désigne Cl, Br, I ou un groupement OH libre ou modifié de manière réactive et fonctionnelle et
R⁵, R⁶ et R⁷ revêtent les significations indiquées selon la revendication 1,
et R⁸ est ensuite éliminé par clivage,
ou
b) un composé de formule IV 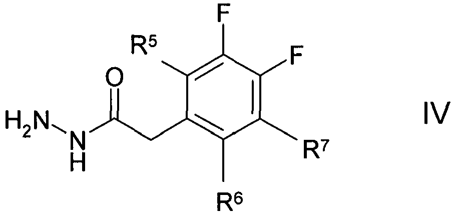 dans lequel
R⁵, R⁶ et R⁷ revêtent les significations indiquées selon la revendication 1,
est réagi avec un composé de formule V 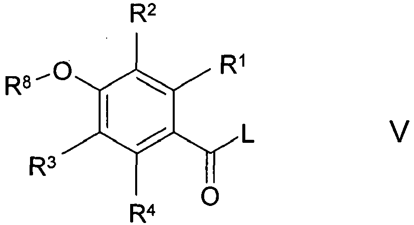 dans lequel
L désigne CI, Br, I ou un groupement OH libre ou modifié de manière réactive et fonctionnelle,
R¹, R², R³ et R⁴ revêtent les significations indiquées selon la revendication 1, et R⁸ désigne un groupement protecteur d'hydroxyle,
et R⁸ est ensuite éliminé par clivage,
ou
c) ils sont libérés depuis l'un de leurs dérivés fonctionnels par un traitement par un agent de solvolyse ou d'hydrogénolyse,
et/ou une base ou un acide de formule I est converti(e) en l'un de ses sels.

10. Médicaments comprenant au moins un composé selon les revendications 1-8 et/ou des sels et stéréoisomères pharmaceutiquement utilisables de celui-ci, y compris des mélanges de ceux-ci en toutes proportions, et éventuellement des excipients et/ou des adjuvants.

11. Utilisation de composés selon les revendications 1-8, et de dérivés, solvates et stéréoisomères pharmaceutiquement utilisables de ceux-ci, y compris des mélanges de ceux-ci en toutes proportions, pour la préparation d'un médicament destiné au traitement ou à la prévention du diabète, de l'obésité, du syndrome métabolique (dyslipidé-mie), de l'hypertonie systémique et pulmonaire, des maladies cardio-vasculaires et rénales, généralement des fibroses et des processus inflammatoires de tout type, du cancer, des cellules tumorales, des métastases tumorales, des coagulopathies, de l'excitabilité neuro-nale, du glaucome, de la cataracte, des infections bactériennes et dans la thérapie anti-infectieuse, pour améliorer les capacités d'apprentissage et l'attention, et pour le traitement et la prophylaxie du stress et du vieillissement cellulaire et pour le traitement de l'acouphène.

12. Utilisation selon la revendication 11, dans laquelle le diabète est le diabète sucré, la néphropathie diabétique, la neuropathie diabétique, l'angiopathie diabétique et la microangiopathie.

13. Utilisation selon la revendication 11, dans laquelle les maladies car-diovasculaires sont les fibroses cardiaques après un infarctus du myocarde, l'hypertrophie cardiaque, l'insuffisance cardiaque et l'artériosclérose.

14. Utilisation selon la revendication 11, dans laquelle les maladies rénales sont la glomérulosclérose, la néphrosclérose, la néphrite, la néphropathie et l'altération de l'excrétion des électrolytes.

15. Utilisation selon la revendication 11, dans laquelle les fibroses et les processus inflammatoires sont la cirrhose hépatique, la fibrose pulmonaire, la pancréatite fibrosante, les rhumatismes et les arthroses, la maladie de Crohn, la bronchite chronique, la fibrose induite par une radiothérapie, la sclérodermie, la mucoviscidose, la cicatrisation et la maladie d'Alzheimer.

16. Médicaments comprenant au moins un composé selon les revendications 1-8 et/ou des sels et stéréoisomères pharmaceutiquement utilisables de celui-ci, y compris des mélanges de ceux-ci en toutes proportions, et au moins un autre composé actif médicamenteux.

17. Ensemble (kit) constitué de conditionnements séparés
(a) d'une quantité efficace d'un composé selon les revendications 1-8 et/ou de sels et stéréoisomères pharmaceutiquement utilisables de celui-ci, y compris des mélanges de ceux-ci en toutes proportions,
et
(b) d'une quantité efficace d'un autre composé actif médicamenteux.
